# EUROPÄISCHE PATENTANMELDUNG

(11) **EP 2 301 523 A1**
(43) Veröffentlichungstag der Anmeldung: **30.03.2011**
(21) Anmeldenummer: 09012046.0
(22) Anmeldetag: 22.09.2009
(51) Int. Cl.: A61K 9/107, A61K 8/06, A61K 8/14

(54) **Galenische Formulierung in kolloidaler Form**

(71) Anmelder: Dr. August Wolff GmbH & Co. KG Arzneimittel, 33611 Bielefeld (DE)
(72) Erfinder: Die Erfindernennung liegt noch nicht vor
(74) Vertreter: Grünecker, Kinkeldey, Stockmair & Schwanhäusser Anwaltssozietät

(57) **Zusammenfassung**

Die vorliegende Erfindung betrifft eine galenische Formulierung in kolloidaler Form, umfassend 0,1 bis 10 Gew. % Linolsäuren; 5 bis 65 Gew. % einer Mischung, umfassend ein Tensid und ein Co-Tensid; sowie 20 bis 95 Gew. % Wasser. Die erfindungsgemäße Formulierung eignet sich besonders zur topischen Applikation auf Haut, Haar, Nägel und/oder Schleimhaut.

## Beschreibung

Die vorliegende Erfindung betrifft eine galenische Formulierung in kolloidaler Form, welche sich besonders zur topischen Applikation auf Haut, Haar, Nägel und/oder Schleimhaut eignet.

### Hintergrund der Erfindung

Linolsäure und deren Derivate (im folgenden kurz als Linolsäuren bezeichnet) sind als Zusätze in Lebensmitteln und pharmazeutischen Zubereitungen bereits seit Jahren bekannt. Als Nahrungsergänzungsmittel wurden Linolsäuren hauptsächlich in Form von Pulvern oder Kapseln angeboten. In pharmazeutischen Präparaten dienen Emulsionen und Lotionen als Vehikel, um dem Körper Linolsäuren zuzuführen. So offenbart beispielsweise die internationale Anmeldung WO 02/070014 A1 eine Öl-in-Wasser-Emulsion zur parenteralen, oralen oder topischen Applikation an Mensch und Tier, die unter anderem Linolsäure enthält.

Emulsionen werden üblicherweise mit Emulgatoren stabilisiert, die eine Mischung von Wasser und Öl ermöglichen. Solche Emulsionen können, zumeist in Form von Salben, Pasten, Gelen oder Cremes, bei Auftragen auf die Haut dazu verwendet werden, eine erweichende oder schützende Wirkung zu erzielen oder sie können Wirkstoffe transportieren. Dabei kann der Emulgator selbst als Transportmittel fungieren oder es wird ein zusätzliches Lipid zugesetzt.

Die bislang bekannten Formulierungen sind jedoch nur geeignet, um Linolsäuren in die oberen Hautschichten eindringen zu lassen. Bei Applikation auf die Haut dringt die Linolsäure, wenn überhaupt, in die oberen Schichten des Stratum Corneums (Hornschicht) ein; die vitalen Schichten der Epidermis werden jedoch kaum erreicht. Mit den bislang bekannten Formulierungen ist eine Beeinflussung des keratinozytären Lipidstoffwechsels bzw. eine Bioverfügbarkeit in tieferen Hautschichten nicht möglich.

### Aufgabe der Erfindung

Aufgabe der Erfindung war es daher, eine Formulierung bereitzustellen, mit der eine bessere Penetration und damit eine bessere Bioverfügbarkeit von Linolsäure erreicht werden kann.

Diese Aufgabe wird gelöst durch eine galenische Formulierung in kolloidaler Form, umfassend:
- 0,1 bis 10 Gew. % Linolsäuren;
- 5 bis 65 Gew. % einer Mischung, umfassend ein Tensid und ein Co-Tensid;
- 20 bis 95 Gew. % Wasser.

Bevorzugte Ausführungsformen sind in den Unteransprüchen und der nun folgenden Beschreibung gegeben.

### Figuren

Figur 1 zeigt die Langzeitstabilität der erfindungsgemäßen galenischen Formulierung ohne und mit Zusatz von Antioxidationsmitteln.
Figur 2 zeigt den Einfluss der Negativ- sowie Positivprobe auf die Perfusion befruchteter Hühnereier.
Figuren 3 und 4 zeigen den Einfluss der erfindungsgemäßen Formulierung mit unterschiedlicher Linolsäurekonzentration auf die Perfusion befruchteter Hühnereier.
Figur 5 zeigt den Vergleich der erfindungsgemäßen Formulierung zu einer herkömmlichen Emulsion nach 30 Minuten Versuchszeit.
Figur 6 zeigt den Vergleich der erfindungsgemäßen Formulierung zu einer herkömmlichen Emulsion nach 300 Minuten Versuchszeit.
Figur 7 zeigt den Vergleich der erfindungsgemäßen Formulierung zu einer herkömmlichen Emulsion nach 1000 Minuten Versuchszeit.

### Detaillierte Beschreibung der Erfindung

Die vorliegende Erfindung betrifft eine galenische Formulierung in kolloidaler Form, umfassend:
- 0,1 bis 10 Gew. % Linolsäuren;
- 5 bis 65 Gew. % einer Mischung, umfassend ein Tensid und ein Co-Tensid;
   und
- 20 bis 95 Gew. % Wasser.

Die erfindungsgemäße Formulierung ist eine galenische Formulierung in kolloidaler Form, die auch als "Mikroemulsionssystem" bezeichnet wird. Mikroemulsionen sind im Gegensatz zu Emulsionen thermodynamisch stabile Systeme. Eine Mikroemulsion ist eine Mischung aus vier Komponenten, nämlich aus zwei normalerweise nicht miteinander mischbaren Flüssigkeiten sowie einem Tensid und einem Co-Tensid. Mikroemulsionen bilden sich spontan, und es bedarf keines Energieaufwandes, der bei der Herstellung von normalen Emulsionen zur Zerteilung der inneren Phase notwendig ist. Zusätzlich bleibt eine Mikroemulsion auch bei längerer Lagerung stabil, während Emulsionen dazu neigen, dass die einzelnen Bestandteile ineinanderfließen. Die galenische Formulierung in kolloidaler Form im Sinne der Erfindung ist eine Mikroemulsion, wie sie oben beschrieben wurde, und unterscheidet sich damit von herkömmlichen Emulsionen, wie sie bislang im Stand der Technik zur Applikation auf Haut, Schleimhaut, Nägel oder Haare verwendet werden.

Die herkömmlichen Emulsionen bilden halbfeste Zubereitungen, während die erfindungsgemäße Mikroemulsion eine flüssige Zubereitung mit geringer Viskosität ist. Die erfindungsgemäßen Formulierungen ermöglichen deshalb eine deutlich verbesserte Penetration für Substanzen, die beispielsweise ein hohes Molekulargewicht haben oder eine extreme hydrophile oder lipophile Löslichkeit haben, und die bislang mittels herkömmlicher Emulsionen nicht in tieferen Körperschichten bioverfügbar waren. Mit der erfindungsgemäßen Formulierung lässt sich Linolsäure bevorzugt in tiefere Schichten der Haut transportieren.

Die erfindungsgemäße galenische Formulierung umfasst eine sogenannte hydrophobe Phase, die auch als lipophile Phase bezeichnet wird und unpolaren Charakter hat, sowie eine hydrophile Phase. Die hydrophobe Phase wird allgemein aus Ölen und Fetten gebildet. Die hydrophile Phase wird im wesentlichen durch Wasser gebildet, dass in der Formulierung in einer Menge von 20 bis 95 Gew. %, bezogen auf die Formulierung, enthalten ist, und kann mit Wasser mischbare Flüssigkeiten und/oder in Wasser lösliche Substanzen umfassen.

Zur Herstellung der Formulierung wird außerdem eine Mischung eines Tensids und Co-Tensids verwendet. Tensid und Co-Tensid werden auch als Emulgator bezeichnet. Tensid und Co-Tensid sind sogenannte "grenzflächenaktive Substanzen", welche die Grenzflächenspannung an der Phasengrenze der hydrophoben und der hydrophilen Phase deutlich senken. Tenside und Co-Tenside haben sowohl einen polaren (hydrophilen) als auch unpolaren (hydrophoben) Teil und bilden sich daher an der Grenzfläche zwischen den Phasen aus.

Die erfindungsgemäße Formulierung umfasst zwei verschiedene Tenside, nämlich ein Tensid und ein Co-Tensid. Als Co-Tensid im Sinne der Erfindung wird das Tensid bezeichnet, das in der Mischung die hydrophoberen bzw. lipophileren Eigenschaften im Vergleich zum Tensid hat. Solche Mischungen aus Tensiden und Co-Tensiden sind dem Fachmann an sich allgemein bekannt.

Als Tensid und Co-Tensid kann prinzipiell jedes an sich bekannte Tensid verwendet werden, umfassend nicht-ionische Tenside wie Alkohole, Ether oder Zucker, anionische Tenside wie Carboxylate, Sulfonate und Sulfate, kationische Tenside, die beispielsweise Ammnoiumgruppen beinhalten, und amphotere Tenside.

Beispiele für nicht-ionische Tenside und Co-Tenside sind Polyalkylenglykolether, Fettalkoholpropoxylate, Alkylglucoside, Alkylpolyglucoside, und Alkylphenoloxylate. Besonders bevorzugt sind Alkylpolyglycoside.

Beispiele für anionische Tenside und Co-Tenside beinhalten Alykcarboxylate, auch Seifen genannt, mit gesättigten oder ungesättigten Alkylresten, Alkylbenzolsulfonate, sowie Fettalkoholsulfate.

Beispiele für kationische Tenside und Co-Tenside beinhalten vor allem quartärere Ammoniumverbindungen, die sich nur in den Alkylresten unterscheiden.

Beispiele für amphotere Tenside und Co-Tenside beinhalten Tenside mit einem polaren Teil, wie einer Carboxylatgruppe, zusammen mit einer quartären Ammoniumgruppe, wie Betaine und Sultaine.

In einer bevorzugten Ausführungsform umfasst die Mischung aus Tensid und Co-Tensid Zuckertenside, beispielsweise Alkylpolyglycoside. Beispiele dafür sind Cetearyl- und Lauryl-Glycoside. Die Tenside, die als Emulgatoren eingesetzt werden, sind daher hydrolysebeständige Tenside auf pflanzlicher Basis, die frei von Polyethylenglycol (PEG) sind. Sie haben ein gutes Schaumbildungsvermögen und eine exzellente dermatologische Verträglichkeit in kosmetischen Produkten. Außerdem sind die eingesetzten Tenside biologisch abbaubar und damit gut umweltverträglich.

Die erfindungsgemäße galenische Formulierung besteht weiterhin vorteilhafterweise im wesentlichen aus dermatologisch sehr gut verträglichen Hilfsstoffen. Weiterhin zeichnet sich die galenische Formulierung durch eine geringe Teilchengröße der dispergierten lipophilen Phase aus und gewährleistet eine gute Verträglichkeit bei Applikation vor allem auf Haut, Schleimhaut, Nägel und Haare.

Die galenische Formulierung umfasst die hydrophile Phase, die vor allem aus Wasser gebildet wird, in einer Menge von 20 bis 95 Gew. %, bevorzugter in einer Menge von 25 bis 90 Gew. %, bezogen auf die Formulierung. Die galenische Formulierung umfasst schließlich von 5 bis 65 Gew. % einer Mischung aus mindestens einem Tensid und Co-Tensid, bevorzugter von 10 bis 50 Gew. %. Tensid und Co-Tensid sind zusammen für die Bildung der oben beschriebenen Mikroemulsion verantwortlich. Das Mischungsverhältnis aus Tensid zu Co-Tensid beträgt 0,5 bis 3,5. Besonders bevorzugt ist ein Mischungsverhältnis von 1 zu 3.

In der Formulierung sind die Linolsäuren bevorzugt in einer Menge von 0,1 bis 5 Gew. %, noch bevorzugter in einer Menge von 0,1 bis 2 Gew. % enthalten. Bei den Linolsäuren handelt es sich in einer Ausführungsform um ein Gemisch aus 9,12-Octadiensäure und 9,11-Octadiensäure.

In einer weiteren bevorzugten Ausführungsform umfasst die hydrophile Phase weiterhin Glykole und/oder Polyole und/oder Puffersysteme. Ein bevorzugtes Polyol ist 1,2-Pentandiol, welches in einer Menge von 1 bis 30 Gew. %, noch bevorzugter von 15 bis 25 Gew. % und ganz besonders bevorzugt in einer Menge von 20 Gew. %, bezogen auf die Formulierung, enthalten ist. Der Anteil des Wassers beträgt 20 bis 95 Gew. %, besonders 50 bis 80 Gew. %, bezogen auf die Formulierung. In einer besonders bevorzugten Ausführungsform umfasst die hydrophile Phase 1,2-Pentadiol und Wasser in einem Verhältnis von 1:10 bis 10:1.

Die Formulierung kann weiterhin Antioxidationsmittel umfassen. Bevorzugte Antioxidationsmittel sind Butylhydroxytoluol, Ascorbylpalmitat und α-Tocopherolacetat. Die Antioxidationsmittel werden in einer Menge von 0,1 bis 2 Gew. %, bezogen auf die Formulierung, eingesetzt.

Die erfindungsgemäße Formulierung kann außerdem Penetrationsverstärker enthalten, wie Alkohole, Terpene und Dimethylsulfoxid.

Die erfindungsgemäße galenische Formulierung eignet sich besonders zur topischen Applikation auf Haut, Hautanhanggebilde, Schleimhäute, Haar, und/oder Nägel.

Nachstehend wird die Erfindung anhand verschiedener Beispiele näher beschrieben. Diese Beispiele dienen lediglich der Erläuterung, die vorliegende Erfindung ist auf diese Beispiele nicht beschränkt.

### Beispiele:

### - Herstellung der galenischen Formulierungen

Zur Herstellung des kolloidalen Mikroemulsionssystems wurden folgende Schritte durchgeführt:
- Einwiegen der Tensid/Cotensidmischung;
- Zugabe der Linolsäure(n);
- Zugabe von 1 ,2-Pentandiol und Wasser;
- Rühren oder Schütteln bis zum klarwerden, evtl. eine kurze Behandlung des kolloidalen Systems mit Ultraschall;
- Analysieren der Mikroemulsion.

### - Analytik

Die Quantifizierung der Linolsäuren erfolgte über die 9,11-Octadiensäure, welche UV-aktiv ist und nur zu vernachlässigbaren Teilen natürlich in der Haut vorkommt.

HPLC-Parameter:
Gerät Agilent Serie 1100
Säule LiChroCART 125-4 LiChrospher 60 RP-select B (5 µm)
Fluent Acetonitril/Wasser/TFA
70/3 0/0,1
Flow 0.2 ml/min
Säulentemperatur 40 °C
Injektions-Volumen 5 µl
Druck 61 bar
Wellenlänge 234 nm
Interner Standard Mometasonfuroat
Lösungen I 2; 5; 10; 20; 50: 100 µg/ml in Methanol
Stammlösung 1000 µg/ml in Methanol
Quantifizierungsgrenze 0.5 µg/ml
Wiederholpräzision 2,27 %
Mittlerer Korrelationskoeffizient (r²) 0,9996

### - Stabilitätsuntersuchungen

Die Lagerstabilität der Linolsäuren im Mikroemulsionssystem wurde über einen Zeitraum von 24 Wochen untersucht. Es wurde folgendes System verwendet, dem als Oxidationsschutz einmal noch zusätzlich Butylhydroxytoluol (0,05 %) und einmal noch zusätzlich Ascorbylpalmitat (0,1 %)als Antioxidationsmittel hinzugesetzt wurde:
Tabelle [1]
   Bestandteil Gew.-%
   Cetearyl-Glucoside 5,00 %
   Lauryl-Glucoside 15,00 %
   1,2-Pentandiol 20,00 %
   Linolsäuren 2,00 %
   Wasser 58,00 %

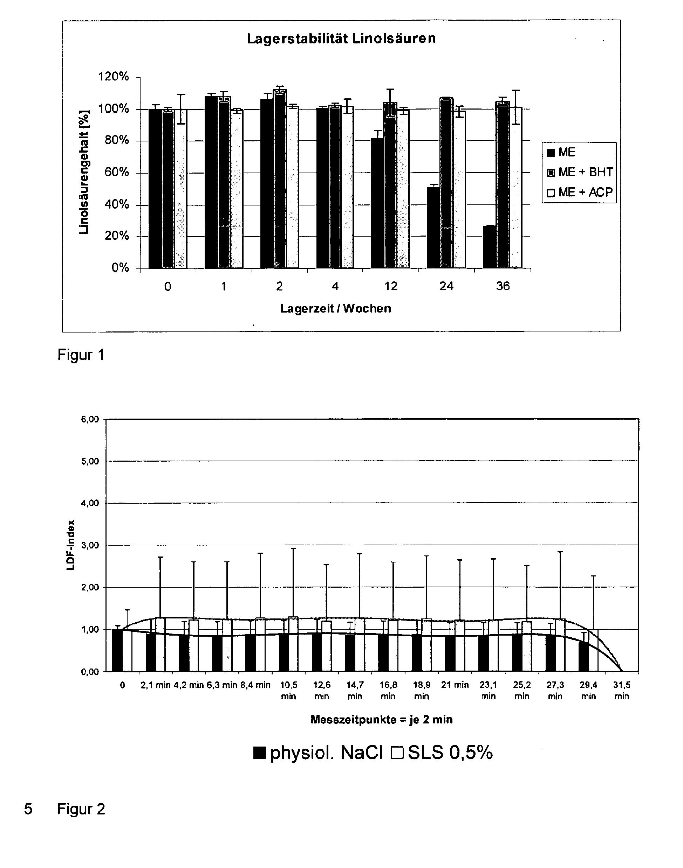

Die Ergebnisse in Abbildung 1 zeigen auf, dass die Linolsäuren über vier Wochen hinweg stabil waren. Die zugesetzten Antioxidationsmittel konnten über diesen Zeitraum keine Verbesserung der Wirkstoffstabilität aufzeigen.

Nach drei Monaten ist jedoch ersichtlich, dass die Systeme mit zugesetztem Oxidationsschutz deutlich stabiler sind. Während ohne Antioxidationsmittel der Linolsäurengehalt nach 12 Wochen auf 82 % bzw. nach 24 Wochen auf 50 % gesunken ist, bleibt der Linolsäurengehalt mit Butylhydroxytoluol (BHT) oder Ascorbylpalmitat (ACP) weiterhin über diesen Zeitraum stabil.

### - HET-CAM Untersuchungen

Mittels der Hen's Egg Test (HET)-Chorio Allantois Membran (CAM)-Methode kann die akute irritierende Wirkung von Substanzen auf das Blutgefaßsystem der CAM unter in vivo Bedingungen untersucht und damit orientierend die Gewebetoxizität von Substanzen für Haut bzw. Schleimhaut abgeschätzt werden.

Es wurde beobachtet, dass sich nach ca. 8-10 Tagen Bebrütung auf der CAM ein dichtes hämovaskuläres Gefäßnetz ausgebildet hatte, welches jedoch keine Nervenfasern enthielt. Die Versuchsdurchführung wird somit als schmerzfrei angesehen, ist als Alternative zum Tierversuch (Draize-Test) international etabliert und akzeptiert und liefert gleichwertige Ergebnisse.

Es wurden natürlich befruchtete Hühnereier der Rasse 'New Hampshire" verwendet, die am Legetag durch das Nutztierwissenschaftliche Zentrum der Martin-Luther-Universität Halle-Wittenberg bereitgestellt wurden. Nach dem Transport wurden die Eier über 8 Tage bei 37 °C und 55 % Luftfeuchtigkeit in Paletten in einem Brutschrank bebrütet. Alle 12 Stunden wurden die Eier gewendet, ausgenommen die letzten 24 Stunden. Nach Ablauf der Brutzeit wurden die Eier einzeln in Polysterolummantelungen überführt, eröffnet und mikrochirurgisch wie folgt unter einer Laminarbox präpariert:

Nach Durchleuchten der Eipole mit einer lichtstarken Taschenlampe wurde ein etwa kreisrundes Loch mit einem Durchmesser von 1,5 cm im Bereich der Luftkammer in den schwächer konvexen Pol des Eis geschnitten. Der durch die Öffnung entstehende Staub wurde vorsichtig mit einem Gummiblasebalg von der Eihaut abgeblasen und die Eihaut mit 37 °C warmer physiologischer Kochsalzlösung benetzt. Durch vorsichtige Präparation mit sterilem mikrochirurgischem Besteck wurde unter einem Lupenmikroskop die äußere Eihaut entfernt und die CAM freigelegt. Ca. 10 % der präparierten Eier waren nicht befruchtet und mussten verworfen werden. Für die Untersuchungen wurden nur Eier verwendet, die ein gut ausgebildetes Gefäßnetz auf der CAM aufwiesen.

Zur Applikation der Testsubstanzen wurden an jeweils 12 Eiern 30 min nach dem Abziehen der Eihaut je 100 µl der Testsubstanz, in diesem Fall die oben aufgeführte Mikroemulsion, und als Vergleich 100 % des Linolsäurengemischs appliziert. Als Negativprobe diente physiologische Kochsalzlösung und als Positivprobe Natriumlaurylsulfat (SLS).

Zur Einschätzung der Perfusion insgesamt wird der Laser-Doppler-Flux (LDF) mit Hilfe der Laser-Doppler-Fluxmetrie und nach definierten Zeitintervallen bis ca. 30min nach Applikation bestimmt. Hierbei wurde die CAM mit monochromatischem, niedrigenergetischem Laserlicht (Helium-Neon Laser mit 632,8 nm - rot) illuminiert. Dabei kollidierten eindringende Photonen an statischen Strukturen und bewegten Blutzellen (Erythrozyten). Bei der Kollision erfolgt eine Reflexion. Allerdings bleibt die Wellenlänge beim Aufprall auf nichtbewegte Strukturen unverändert; hingegen resultiert beim Aufprall auf bewegte Zellen oder Partikeln eine proportional zur Geschwindigkeit verschobene Änderung der Wellenlänge (Doppler-Shift).

Die reflektierten Photonen werden durch ein Lichtleitkabel aufgenommen und zu einem Photodetektor geleitet. Durch einen Mikroprozessor werden die Signale so berechnet, dass sie eine entsprechend des zeitlichen Verlaufs orientierte Amplitude ergeben. die direkt proportional zur Blutperfusion (Flux) ist. Um die biologisch exakte Proportionalität zwischen der Anzahl der Erythrozyten und dem Blutvolumen zu gewährleisten, muss das Laser-Doppler-Signal in einem linearen Verhältnis zum Volumen-Fließgeschwindigkeits-Produkt des Blutes im gemessenen Volumen stehen. Deshalb muss diese Beziehung und das gemessene Gewebevolumen durch mehrere Faktoren mathematisch korrigiert werden, die die Eigenschaften des Laserlichtes, des optischen Systems und dessen Kopplung mit dem Gewebe, die Bauweise des Photodetektors und eingebauter Filtersysteme, der digitalen Bearbeitung des Gewebes und der Verteilung des lokalen Blutflusses berücksichtigen.

Zur besseren Vergleichbarkeit und zur Korrektur von Perfusionsdifferenzen durch Unterschiede im Gefäßdurchmesser, Blutdruck oder/und Gefäßanzahl wird jeweils ein Index (LDF-Index) berechnet der den Messwert zum Zeitpunkt x ins Verhältnis zum Baseline-Wert vor Beginn des Einflussfaktors setzt. Dabei wird der Wert des LDF unmittelbar vor Applikation der Testsubstanz (LDF 0) und der Wert des LDF nach Applikation der Testsubstanz (LDFx) ins Verhältnis gesetzt. Die LDF-Indices werden in Abhängigkeit von der Konzentration der jeweiligen Testsubstanz bzw. der Applikationszeiten graphisch dargestellt und statistisch ausgewertet.

Die Lyse sowie das Transparentwerden von Gefäßen nach 5 min wurden als Membranverfärbung (MD) gewertet. Dabei wurden drei Kategorien unterschieden: Vereinzelte transparente Kapillarabschnitte wurden als leichte, die Transparenz von ganzen Kapillaren als mittlere und das Auftreten von Lysen als schwere MD bewertet. Die Hämorrhagie (HR) wurde entsprechend des Ausmaßes von Erythrozytenextravasaten ebenfalls nach einer Applikationszeit von 5 min in 3 Kategorien halbquantitativ beurteilt. Einzelne Kapillarblutungen bei morphologisch intakten Kapillaren wurden als leichte, multiple Kapillarblutungen bei morphologisch intakten Kapillaren als mittlere und morphologisch geschädigte Kapillaren mit Kapillarblutung oder Massenblutung als schwere HR eingestuft.

Nach den Versuchen wurden alle Eier über 48 Stunden im Tiefkühlschrank bei -20 °C abgetötet und anschließend hygienisch über den Krankenhausmüll entsorgt.

Figur 2 zeigt den Einfluss der Negativprobe (physiologische NaCl) sowie der Positivprobe (SLS) auf die Perfusion der befruchteten Hühnereier. Dargestellt sind Mittelwert + Standardabweichung (n = 12).

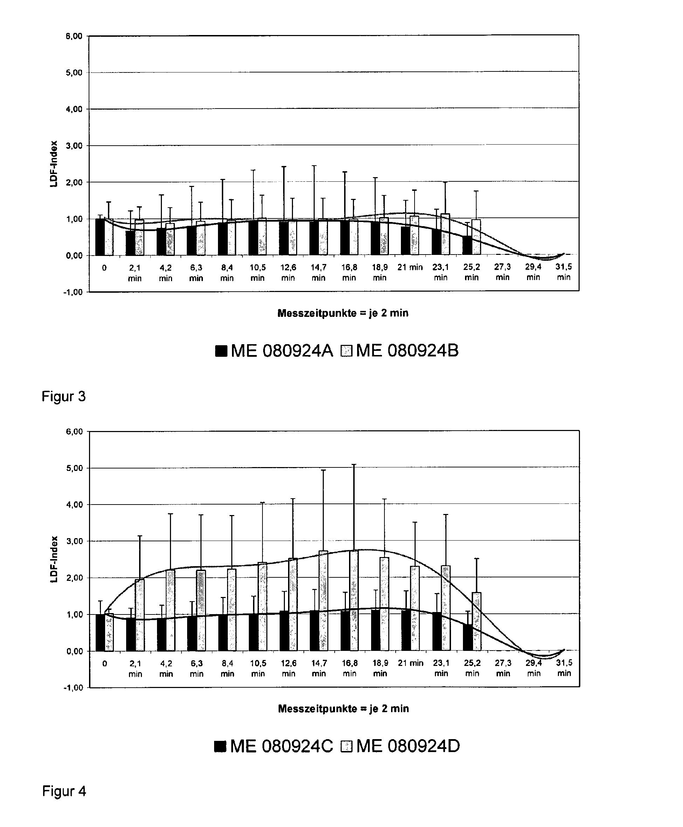

Figur 3 zeigt den Einfluss der Mikroemulsion mit 0,5 %iger Linolsäurenkonzentration (ME 080924A) sowie der Mikroemulsion mit 1 %iger Linolsäurenkonzentration (ME 080924B) auf die Perfusion der befruchteten Hühnereier. Dargestellt sind Mittelwert ± Standardabweichung (n = 12).

Figur 4 zeigt den Einfluss der Mikroemulsion mit 2 %iger Linolsäurenkonzentration (ME 080924C) sowie der Mikroemulsion mit 5 %iger Linolsäurenkonzentration (ME 080924D) auf die Perfusion der befruchteten Hühnereier. Dargestellt sind Mittelwert ± Standardabweichung (n = 12).

Die Untersuchung der Negativ- und Positivkontrollen ergab wie erwartet für die physiologische Kochsalzlösung keinen Hinweis für einen irritativen Effekt, während für Natriumlaurylsulfat eine deutliche Steigerung der Perfusion als Ausdruck eines irritativen Effektes erkennbar war.

Die erfindungsgemäßen Mikroemulsionen hingegen zeigten bis auf das System mit 5 %igem Linolsäurengehalt keine Hinweise für eine irritative Wirkung oder einen vasoaktiven Effekt. Lediglich bei der Mikroemulsion mit 5 % Linolsäuren kam es zu einer Steigerung der Perfusion als Ausdruck eines irritativen Effektes.

**Tab. [2] Visueller Score der HET-CAM Untersuchungen**

| Präparat | Vital | HR | MD | | | Gesamt |
|---|---|---|---|---|---|---|
| | | | leicht | mittel | schwer | |
| Physiol. NaCl | 12 | 0 | 0 | 0 | 0 | 12 |
| SLS | 12 | 3 | 9 | 1 | 2 | 12 |
| ME 080924 A | 12 | 0 | 0 | 0 | 0 | 12 |
| ME 080924 B | 12 | 0 | 2 | 0 | 0 | 12 |
| ME 080924 C | 12 | 0 | 3 | 0 | 0 | 12 |
| ME 080924 D | 12 | 9 | 6 | 3 | 3 | 12 |

Bis zu einer Konzentration von 2 % Linolsäuren findet sich kein Hinweis für eine irritative Wirkung. Die Zubereitungen ME 080924 A (0,5 % Linolsäuren) -B (1,0 % Linolsäuren) und -C (2 % Linolsäuren) können als hautverträglich im Sinne des Testes gelten. Die ME 080924D (5 % Linolsäuren) muss als irritativ bewertet werden. Systeme mit therapeutischen Linolsäurenkonzentrationen sind somit als unbedenklich zu bewerten.

### - Penetrationsuntersuchungen

Die Penetration der Linolsäuren aus der entwickelten Mikroemulsion wurde anhand der bekannten Diffusionszelle nach FRANZ (Crown Glass Company, Somerville, New York, USA) untersucht. Als Vergleichspräparat wurde Linola Fett untersucht, eine Emulsion, deren Linolsäurenkonzentration auf 2 % erhöht wurde, um einen direkten Vergleich mit der erfindungsgemäßen Mikroemulsion zu erhalten. Für die Penetrationsuntersuchung wurden Humanhautstücke verwendet, die von Mammareduktionspiastiken stammten.

Der Aufbau der Diffusionszelle nach FRANZ ist wie folgt: Das durch einen Glasring und eine Metallklammer auf der Filtergaze positionierte Hautstück steht von der dermalen Seite aus in Kontakt mit der Akzeptorflüssigkeit, in diesem Fall ein Phosphatpuffer pH 7,4. Durch eine Glasabdeckung wird das System vor Verdunstungsverlusten während des Versuches geschützt.

Es wurden jeweils ca. 20 µl Mikroemulsion oder ca. 20 mg Linola Fett (2 % Linolsäuren) auf ein entsprechendes Hautstück aufgetragen und gleichmäßig verteilt. Es wurden drei Spenderhäute zu drei unterschiedlichen Versuchszeiten verwendet. Anschließend wurde das präparierte Hautstück auf der Gaze der auf 32°C temperierten Diffusionszelle platziert und dort 30, 300 oder 1000 Minuten belassen. Nach Ablauf der Versuchszeit wurde die überschüssige Formulierung auf der Hautoberfläche mit einem Tupfer entfernt.

Das Hautstück diente zur Gewinnung von drei Stanzbiopsien (Kromayer-Stanze, Durchmesser 6 mm; Stiefel Laboratorium GmbH, Offenbach), aus denen mit Hilfe eines Gefriermikrotoms (Jung, Heidelberg) horizontale Schnitte angefertigt wurden. Die einzelnen Schnitte sind in Tabelle 3 aufgeführt.

**Tab. [3] Probenaufbereitung der Penetrationsuntersuchung von Linolsäuren (Anzahl und Dicke der horizontalen Schnitte gewonnen aus einer Stanzbiopsie)**

| **Hautprobe** | **Schnittanzahl und Schichtdicke** |
|---|---|
| Stratum Corneum (SC) | 10 µm |
| lebende Epidermis (EP) | 4 Schnitte a 20 µm |
| Dermis 1 (DR 1) | 5 Schnitte à 40 µm |
| Dermis 2 (DR 2) | 5 Schnitte à 40 µm |
| Dermis 3 (DR 3) | 5 Schnitte à 40 µm |
| Dermis 4 (DR 4) | 5 Schnitte à 40 µm |
| Dermis 5 (DR 5) | 5 Schnitte à 40 µm |

Die zur gleichen Hautschicht gehörenden Schnitte aller drei Stanzen wurden zu einer Probe zusammengefasst. Der zum Abwischen der überschüssigen Formulierung verwendete Tupfer wurde zur Extraktion mit 5,0 ml Methanol 12 h Stunden geschüttelt. Die einzelnen Hautschnitte wurden mit 300-500 µl Methanol, je nach erwarteter Wirkstoffmenge, versetzt und eine Stunde geschüttelt. Dem Methanol war vor der Extraktion der Hautproben Mometasonfuroat in einer Konzentration von 5 µg/ml zugesetzt worden. Dieses diente als interner Standard. Anschließend wurden die Proben über Nacht im Kühlschrank aufbewahrt und am nächsten Tag erneut eine Stunde geschüttelt. Die Analytik erfolgte ebenfalls mit HPLC-UV.

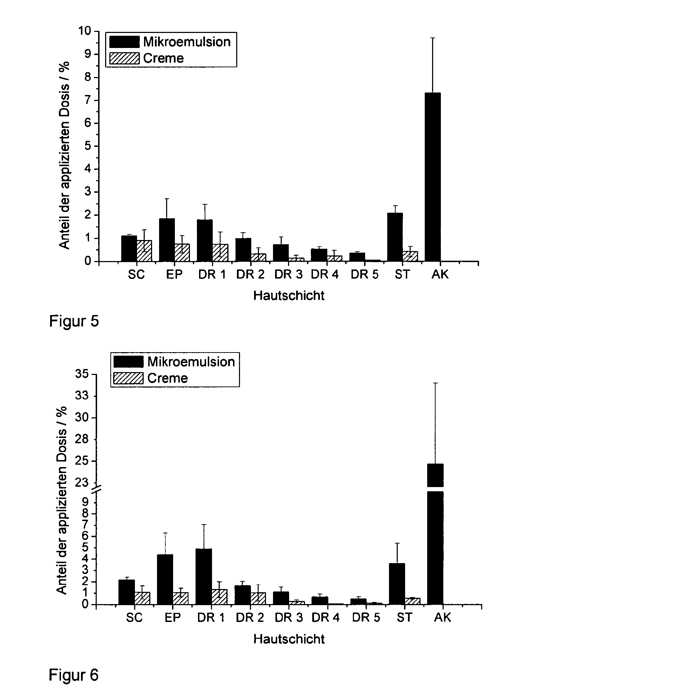

Aus den Figuren 5 bis 7 ist ersichtlich, dass zu allen drei Versuchszeiten höhere Linolsäurengehalte aus der Mikroemulsion in die Haut penetriert sind. Schon nach nur 30 Minuten finden sich in der Epidermis beim Mikroemulsionssystem mit 1,84 % mehr als doppelt soviel des Anteils der applizierten Dosis an Linolsäuren verglichen zu herkömmlichem Linola Fett mit nur 0,74 % des Anteils der applizierten Dosis.

Figur 5 zeigt den Vergleich der erfindungsgemäßen Mikroemulsion und Linola Fett Linolsäuren nach einer Penetration nach 30 Minuten Versuchszeit, mit den Anteilen der applizierten Dosis (SC stratum corneum; EP = lebende Epidermis; DR1 = Dermis 1; DR2 = Dermis 2; DR3 = Dermis 3; DR4 = Dermis 4; DR5 - Dermis 5; ST = Stumpf; AK = Akzeptorkompartiment).

Figur 6 zeigt den Vergleich der erfindungsgemäßen Mikroemulsion und Linola Fett Linolsäuren nach einer Penetration nach 300 Minuten Versuchszeit, angegeben in Anteilen der applizierten Dosis (SC = Stratum Corneum; EP = lebende Epidermis; DR1 = Dermis 1; DR2 = Dermis 2: DR3 = Dermis 3; DR4 = Dermis 4; DR5 = Dermis 5; ST = Stumpf; Ak = Akzeptorkompartiment).

Nach 1000 Minuten ist dieser Unterschied noch deutlicher. Linola Fett erreicht nur einen Anteil von 1,73 %, während bei der Mikroemulsion 7,37 % in der Epidermis zu finden sind. Der Linolsäuregehalt steigt bei der Mikroemulsion besonders in der Epidermis und in der Dermis 1 mit längerer Versuchszeit an.

Linola Fett ist, wie in Tabelle 4 ersichtlich, nicht in der Lage, den Wirkstoffgehalt in diesen Hautschichten mit längerer Penetrationszeit stark zu erhöhen. In den unteren Dermisschichten bleibt bei beiden Formulierungen die Linolsäurenkonzentration zu allen drei Inkubationszeiten weitestgehend konstant, was für eine Kumulation des Wirkstoffes in der Epidermis spricht. Die vorliegenden Ergebnisse zeigen, dass es möglich ist, mit Hilfe des erfindungsgemäßen Mikroemulsionssystems in kurzer Zeit hohe Raten an Linolsäuren in die Haut penetrieren zu lassen.

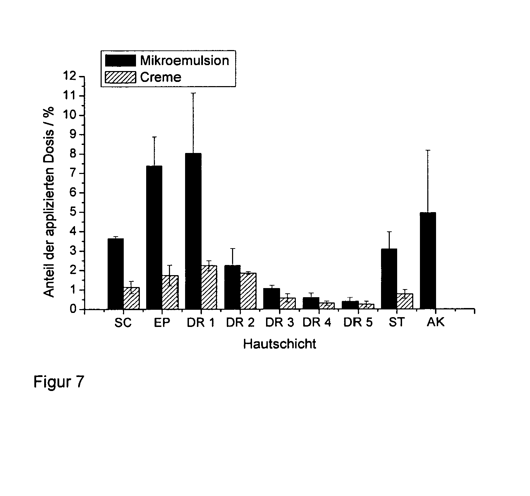

Figur 7 zeigt den Vergleich zwischen der erfindungsgemäßen Mikroemulsion und Linola Fett Linolsäuren nach einer Penetration nach 1000 Minuten Versuchszeit, angegeben in Anteilen der applizierten Dosis (SC = Stratum Corneum; EP = lebende Epidermis; DR1 = Dermis 1; DR2 = Dermis 2; DR3 = Dermis 3; DR4 = Dermis 4; DR5 = Dermis 5; ST = Stumpf; AK = kzeptorkompartiment).

Im Vergleich zum Standardvehikel war die Mikroemulsion zu allen drei Versuchzeiten der Linola Fett Creme überlegen. Von Vorteil ist, dass sich die Linolsäuren auch bei längeren Versuchszeit nicht in den tiefen Dermisschichten anreichern, sondern eine Erhöhung des Wirkstoffgehaltes im Zielkompartiment Epidermis erfolgt.

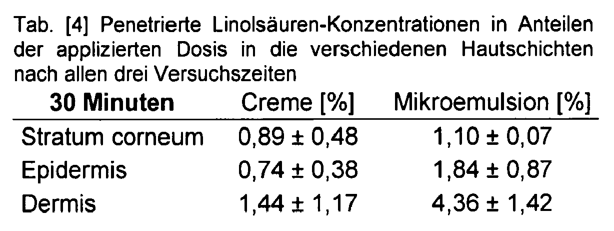

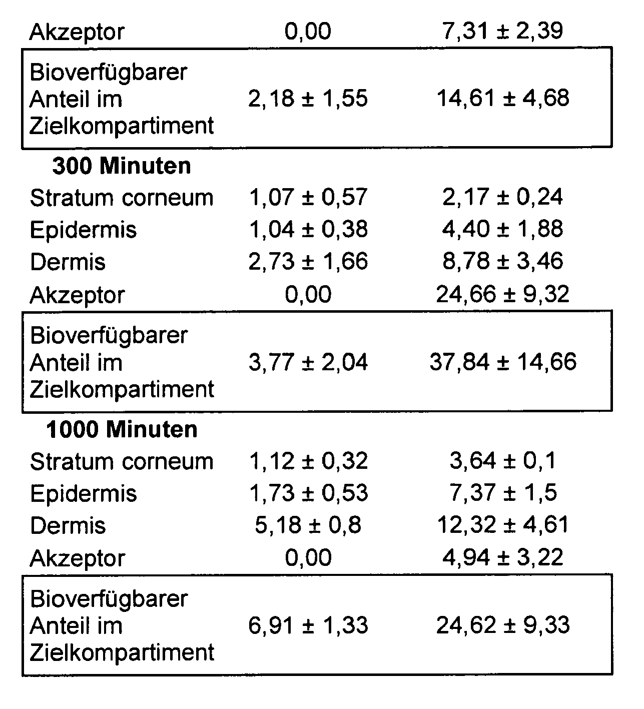

**Tab. [5] Penetrierte Linolsäuren-Konzentrationen in µM in die angegebene Hauttiefe nach allen drei Versuchszeiten**

| **30 Minuten** | | |
|---|---|---|
| Hauttiefe [µm] | Creme [µM] | Mikroemulsion [µM] |
| 10 | 4125,91 ± 1235,84 | 5319,25 ± 216,68 |
| 90 | 358,05 ± 156,55 | 991,51 ± 383,52 |
| 290 | 141,11 ± 88,14 | 385,84 ± 120,1 |
| 490 | 60,86 ± 42,64 | 213,45 ± 43,95 |
| 690 | 25, 09 ± 18,70 | 153,50 ± 59,06 |
| 890 | 42,71 ± 37,27 | 111,45 ± 17,51 |
| 1090 | 9,64 ± 0,95 | 75,38 ± 10,07 |

| **300 Minuten** | | |
|---|---|---|
| 10 | 3610,65 ± 1523,92 | g302,52 ± 732,85 |
| 90 | 508 ± 149,84 | 2370,98 ± 826,55 |
| 290 | 252,9 ± 106,46 | 1057,45 ± 380,51 |
| 490 | 195,81 ± 111,22 | 353,13 ± 72,25 |
| 690 | 51,36 ± 19,71 | 234,65 ± 78,68 |
| 890 | 9, 94 ± 5,17 | 143,19 ± 45, 99 |
| 1090 | 20,70 ± 13,47 | 103,98 ± 31,79 |

| **1000 Minuten** | | |
|---|---|---|
| 10 | 4278,53 ± 804, 9 | 12900,1 ± 310, 21 |
| 90 | 856,28 ± 201,81 | 3970,14 ± 661, 88 |
| 290 | 443,02 ± 47,65 | 1731,15 ± 547,76 |
| 490 | 366,47 ± 6,47 | 486,12 ± 153,77 |
| 690 | 112,9 ± 34,56 | 229,26 ± 28,96 |
| 890 | 59,82 ± 18,66 | 125,42 ± 43,45 |
| 1090 | 47,61 ± 24,12 | 84,97 ± 37,72 |

Die erfindungsgemäße galenische Formulierung basiert auf einer Mikroemulsion und hat deutlich verbesserte Penetrationseigenschaften verglichen mit herkömmlichen Emulsionen. Linolsäuren können bei beispielsweise Applikation auf die Haut in deutlich tiefere Hautschichten eindringen, wodurch die Bioverfügbarkeit deutlich erhöht werden kann. Die erfindungsgemäße Formulierung eignet sich ganz besonders zur topischen Applikation auf Haut, Hautanhanggebilde, Schleimhäute, Haar und/oder Nägel.

## Patentansprüche

1. Galenische Formulierung in kolloidaler Form, umfassend:
- 0,1 bis 10 Gew. % Linolsäuren;
- 5 bis 65 Gew. % einer Mischung, umfassend ein Tensid und ein Co-Tensid;
und
- 20 bis 95 Gew. % Wasser.

2. Galenische Formulierung nach Anspruch 1, umfassend 0,1 bis 5 Gew. % Linolsäuren.

3. Galenische Formulierung nach Anspruch 1, umfassend 0,1 bis 2 Gew. % Linolsäuren.

4. Galenische Formulierung nach mindestens einem der Ansprüche 1 bis 3, worin die Mischung aus Tensid und Co-Tensid Alkylpolyglycoside umfasst.

5. Galenische Formulierung nach mindestens einem der Ansprüche 1 bis 4, worin das Massenverhältnis von Tensid zu Co-Tensid von 0,25 bis 3,5 ist.

6. Galenische Formulierung nach mindestens einem der Ansprüche 1 bis 5, worin das Massenverhältnis von Tensid zu Co-Tensid 1 bis 3 ist.

7. Galenische Formulierung nach mindestens einem der Ansprüche 1 bis 6, welche weiterhin Glykole und/oder Polyole umfasst.

8. Galenische Formulierung nach mindestens einem der Ansprüche 1 bis 7, welche weiterhin einen Puffer umfasst.

9. Galenische Formulierung nach Anspruch 7, worin die hydrophile Phase 1,2-Pentandiol umfasst.

10. Galenische Formulierung nach Anspruch 9, worin die hydrophile Phase 1,2-Pentandiol zu Wasser in einem Verhältnis von 1:10 bis 10:1 umfasst.

11. Galenische Formulierung nach mindestens einem der Ansprüche 1 bis 10, worin die Formulierung weiterhin ein Antioxidationsmittel umfasst.

12. Galenische Formulierung nach Anspruch 11, worin das Antioxidationsmittel ausgewählt ist aus der Gruppe, bestehend aus Butylhydroxytoluol und Ascorbylpalmitat.

13. Verwendung der galenischen Formulierung nach mindestens einem der Ansprüche 1 bis 12 zur topischen Applikation auf Haut, Haar, Nägel und/oder Schleimhaut.
